# EUROPEAN PATENT APPLICATION

(11) **EP 4 645 324 A1**
(43) Date of publication of application: **05.11.2025**
(21) Application number: 23911814.4
(22) Date of filing: 18.12.2023
(51) Int. Cl.: G16C 60/00

(54) **PREDICTION DEVICE, PREDICTION METHOD, AND PREDICTION PROGRAM**

(30) Priority: 28.12.2022 JP 2022211219
(71) Applicant: Resonac Corporation, Tokyo 105-7325 (JP)
(72) Inventor: OKANO, Yu, Tokyo 105-7325 (JP); KANESHITA, Takeshi, Tokyo 105-7325 (JP); TAKEMOTO, Shimpei, Tokyo 105-7325 (JP)
(74) Representative: Strehl & Partner mbB
(86) International application number: PCT/JP2023/045251
(87) International publication number: WO 2024/143033

(57) **Abstract**

One object is to easily understand the degree of impact on a phase ratio caused by a modification of a material composition. A prediction device includes a storage unit configured to store a trained model that is trained using training data in which a material composition of a material to be trained, and a phase ratio of the material to be trained at each temperature within a predetermined temperature range, are associated with each other, wherein a value predicted for a phase specified in advance is partially differentiated using a material composition of a material to be predicted at each temperature, when the phase ratio of the material to be predicted at each temperature within the predetermined temperature range is predicted by inputting the material composition of the material to be predicted to the trained model, and each area specified based on each component and based on each temperature is displayed in a display mode according to the partially differentiated value, in a heat map in which each component of the material to be predicted is arranged on a first axis and each temperature within the predetermined temperature range is arranged on a second axis.

## Description

### TECHNICAL FIELD

The present disclosure relates to prediction devices, prediction methods, and prediction programs.

### BACKGROUND ART

When designing materials, such as alloys or the like, it is important to calculate a phase ratio in a thermodynamic equilibrium state over a predetermined temperature range, and a surrogate model for predicting the relevant phase ratio based on material composition information (in the case of alloys, a weight % of additive elements) is being developed.

The phase ratio within the predetermined temperature range is correlated to material properties. For this reason, when designing the material using the model described above, it is important from a viewpoint of efficiently designing the material that a designer understands in advance how the material composition should be modified in order to obtain a phase ratio close to a desired phase ratio (in the case of an alloy, which phase of which temperature range can be affected by increasing or decreasing the weight % of which additive element).

### PRIOR ART DOCUMENTS

### PATENT DOCUMENTS

Patent Document 1: International Publication Pamphlet No. WO 2020/090617

### DISCLOSURE OF THE INVENTION

### PROBLEM TO BE SOLVED BY THE PRESENT INVENTION

In contrast, if an operation of predicting the phase ratio within a predetermined temperature range while successively increasing or decreasing the weight % of all the additive elements and comparing the predicted phase ratio with the phase ratio before the modification, for example, a degree of impact of the weight % of each additive element on the phase ratio can be understood in advance. However, according to such a method, if the alloy or the like includes a large number of additive elements, there is a problem in that a work load on the designer increases and it is difficult for the designer to understand the degree of impact on the phase ratio.

One object of the present disclosure is to facilitate understanding of the degree of impact on the phase ratio caused by a modification of a material composition.

### MEANS OF SOLVING THE PROBLEM

According to a first aspect of the present disclosure, a prediction device includes:
a storage unit configured to store a trained model that is trained using training data in which a material composition of a material to be trained, and a phase ratio of the material to be trained at each temperature within a predetermined temperature range, are associated with each other, the trained model being configured to predict a phase ratio at an (i+1)-th temperature using phase ratios predicted by the trained model for temperatures to an i-th temperature (i is an integer greater than or equal to 1) within the predetermined temperature range, wherein:
a value predicted for a phase specified in advance is partially differentiated using a material composition of a material to be predicted at each temperature, when the phase ratio of the material to be predicted at each temperature within the predetermined temperature range is predicted by inputting the material composition of the material to be predicted to the trained model, and
each area specified based on each component and based on each temperature is displayed in a display mode according to the partially differentiated value, in a heat map in which each component of the material to be predicted is arranged on a first axis and each temperature within the predetermined temperature range is arranged on a second axis.

According to a second aspect of the present disclosure, in the first aspect, an architecture capable of processing time series data which are data at predetermined time intervals is applied to the trained model, and the trained model predicts a phase ratio for each predetermined temperature interval based on the material composition of the material to be predicted.

According to a third aspect of the present disclosure, in the second aspect, the trained model is any one of an RNN, a bidirectional RNN, a Seq2Seq, a Seq2Seq with Attention mechanism, a GRU, an LSTM, and a Transformer.

According to a fourth aspect of the present disclosure, in the third aspect, the trained model includes:
an encoder unit configured to output a feature, in response to an input of the material composition of the material to be predicted, and
a decoder unit configured to predict the phase ratio at the (i+1)-th temperature, in response to an input of the output feature and predicted phase ratios for the temperatures to the i-th temperature.

According to a fifth aspect of the present disclosure, in any one of the first through fourth aspects, the phase ratio is a phase ratio in a thermodynamic equilibrium state.

According to a sixth aspect of the present disclosure, in any one of the first through fifth aspects:
the value predicted for the phase specified in advance is partially differentiated using the material composition of the material to be predicted, when predicting the phase ratio at the (i+1)-th temperature, and
each area specified based on each component and the (i+1)-th temperature in the heat map is displayed in a display mode according to the partially differentiated value.

According to a seventh aspect of the present disclosure, a prediction method to be implemented in a computer for a trained model that is trained using training data in which a material composition of a material to be trained, and a phase ratio of the material to be trained at each temperature within a predetermined temperature range, are associated with each other, the trained model being configured to predict a phase ratio at an (i+1)-th temperature using phase ratios predicted by the trained model for temperatures to an i-th temperature (i is an integer greater than or equal to 1) within the predetermined temperature range, the prediction method including the steps of:
predicting, by the computer, a value predicted for a phase specified in advance is partially differentiated using a material composition of a material to be predicted at each temperature, when the phase ratio of the material to be predicted at each temperature within the predetermined temperature range is predicted by inputting the material composition of the material to be predicted to the trained model, and
displaying, by the computer, each area specified based on each component and based on each temperature in a display mode according to the partially differentiated value, in a heat map in which each component of the material to be predicted is arranged on a first axis and each temperature within the predetermined temperature range is arranged on a second axis.

According to an eighth aspect of the present disclosure, a prediction program for causing a computer to implement a trained model that is trained using training data in which a material composition of a material to be trained, and a phase ratio of the material to be trained at each temperature within a predetermined temperature range, are associated with each other, the trained model being configured to predict a phase ratio at an (i+1)-th temperature using phase ratios predicted by the trained model for temperatures to an i-th temperature (i is an integer greater than or equal to 1) within the predetermined temperature range, the prediction program causing the computer to perform the steps of:
predicting a value predicted for a phase specified in advance is partially differentiated using a material composition of a material to be predicted at each temperature, when the phase ratio of the material to be predicted at each temperature within the predetermined temperature range is predicted by inputting the material composition of the material to be predicted to the trained model, and
displaying each area specified based on each component and based on each temperature in a display mode according to the partially differentiated value, in a heat map in which each component of the material to be predicted is arranged on a first axis and each temperature within the predetermined temperature range is arranged on a second axis.

### EFFECTS OF THE PRESENT INVENTION

According to the present disclosure, it is possible to facilitate understanding of the degree of impact on the phase ratio caused by a modification of a material composition.

### BRIEF DESCRIPTION OF THE DRAWINGS

[FIG. 1] FIG. 1 is a diagram illustrating an example of a system configuration of a prediction system and examples of functional configurations of a training device and a prediction device.
[FIG. 2] FIG. 2 is a diagram illustrating examples of hardware configurations of the training device and the prediction device.
[FIG. 3] FIG. 3 is a diagram illustrating a configuration of training data and specific examples of input data and ground truth.
[FIG. 4] FIG. 4 is a diagram illustrating an example of a functional configuration of a training data generation unit.
[FIG. 5] FIG. 5 is a diagram illustrating an example of a functional configuration of a training unit.
[FIG. 6] FIG. 6 is a first diagram illustrating an example of an operation of the training unit.
[FIG. 7] FIG. 7 is a second diagram illustrating the example of the operation of the training unit.
[FIG. 8] FIG. 8 is a diagram illustrating an example of a method of calculating a loss by a loss function calculation unit.
[FIG. 9] FIG. 9 is a flow chart illustrating a flow of a training process.
[FIG. 10] FIG. 10 is a diagram illustrating an example of a functional configuration of a prediction unit.
[FIG. 11A]FIG. 11A is a first diagram illustrating an example of an operation of the prediction unit.
[FIG. 11B]FIG. 11B is a second diagram illustrating the example of the operation of the prediction unit.
[FIG. 11C]FIG. 11C is a third diagram illustrating the example of the operation of the prediction unit.
[FIG. 12] FIG. 12 is a flow chart illustrating a flow of a prediction process.
[FIG. 13] FIG. 13 is a diagram illustrating an example of a predicted phase ratio.
[FIG. 14] FIG. 14 is a diagram illustrating an example of a heat map.
[FIG. 15A]FIG. 15A is a first diagram illustrating an example of a verification result.
[FIG. 15B]FIG. 15B is a second diagram illustrating the example of the verification result.
[FIG. 15C]FIG. 15C is a third diagram illustrating the example of the verification result.
[FIG. 15D]FIG. 15D is a fourth diagram illustrating the example of the verification result.
[FIG. 15E]FIG. 15E is a fifth diagram illustrating the example of the verification result.
[FIG. 15F]FIG. 15F is a sixth diagram illustrating the example of the verification result.
[FIG. 15G]FIG. 15G is a seventh diagram illustrating the example of the verification result.
[FIG. 15H]FIG. 15H is an eighth diagram illustrating the example of the verification result.

### MODE OF CARRYING OUT THE INVENTION

Hereinafter, each embodiment will be described with reference to the accompanying drawings. In the present specification and drawings, constituent elements having substantially the same functional configuration are designated by the same reference numerals, and a redundant description thereof will be omitted.

### [First Embodiment]

### <System Configuration of Prediction System, and Functional Configurations of Training Device and Prediction Device>

First, a system configuration of a prediction system including a prediction device according to a first embodiment, and functional configurations of a training device and the prediction device included in the relevant prediction system will be described. In the present embodiment, the prediction system predicts a phase ratio at each temperature within a predetermined temperature range (phase ratio in a thermodynamic equilibrium state, hereinafter simply referred to as a "phase ratio"), based on material composition information.

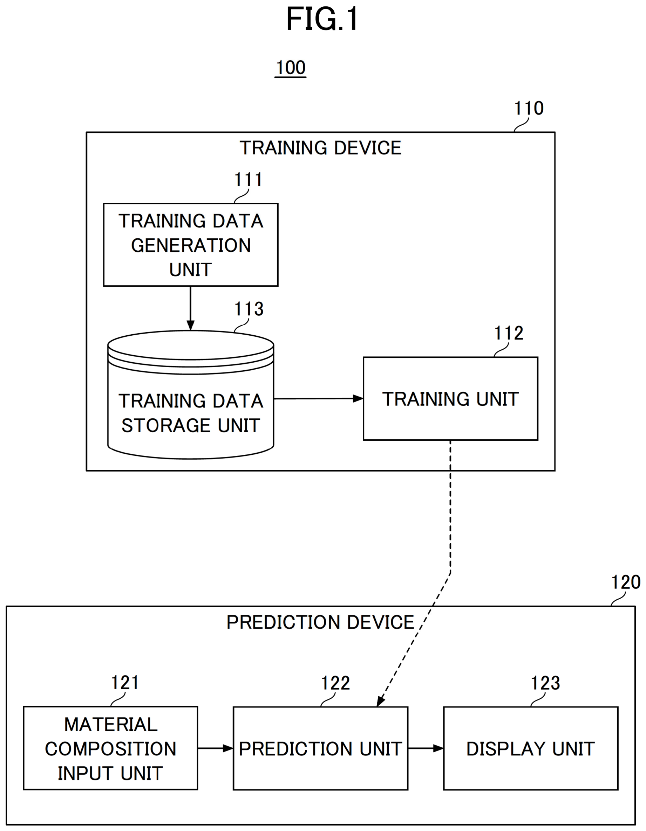

FIG. 1 is a diagram illustrating an example of the system configuration of the prediction system and examples of functional configurations of the training device and the prediction device. As illustrated in FIG. 1, a prediction system 100 includes a training device 110, and a prediction device 120.

A training program is installed in the training device 110, and the training device 110 functions as a training data generation unit 111, and a training unit 112 by executing the relevant training program.

The training data generation unit 111 generates training data for training a prediction model (surrogate model), and stores the generated training data in the training data storage unit 113. In the present embodiment, the training data storage unit 113 stores, as the training data for training the prediction model:
- a plurality of material composition information of different combinations; and
- a phase ratio of materials having the respective material composition information at each temperature within a predetermined temperature range,
in association with each other.

The training unit 112 reads the training data from a training data storage unit 113, and trains the prediction model using the read training data. The training unit 112 trains the prediction model so that output data in a case where the material composition information stored in the training data is input to the prediction model approaches the phase ratio at each temperature within the predetermined temperature range stored in association with the training data. Accordingly, the training unit 112 generates a trained prediction model. In addition, the training unit 112 stores the generated trained prediction model in a storage unit of the prediction unit 122 of the prediction device 120.

In the present embodiment, the training unit 112 implements, as the prediction model, an architecture capable of processing time series data which are data at predetermined time intervals (generally, an architecture used for natural language processing or the like), such as:
- Seq2Seq with Attention mechanism; or
- Transformer.
Thus, according to the relevant prediction model, when the material composition information stored in the training data is input thereto, it is possible to successively output the output data corresponding to the phase ratio at each temperature within the predetermined temperature range.

The expression "successively output the output data corresponding to the phase ratio at each temperature" means that the prediction model outputs the output data corresponding to the phase ratio at an (i+1)-th temperature, using ground truth stored in the training data for the temperatures to the i-th temperature, for example. In this case, i is an integer greater than or equal to 1.

Accordingly, the training unit 112 has a configuration of successively outputting the output data corresponding to the phase ratio at each temperature (a configuration for processing the output data corresponding to the phase ratio at each temperature as time series data). Thus, the training unit 112 can perform a training which reflects the output data corresponding to the phase ratio in an adjacent temperature region.

A prediction program is installed in the prediction device 120, and the prediction device 120 functions as a material composition input unit 121, a prediction unit 122, and a display unit 123 by executing the relevant prediction program.

In a case where material composition information of a material to be predicted is input, the material composition input unit 121 receives the material composition information and notifies the prediction unit 122 of the material composition information.

The prediction unit 122 stores the trained prediction model trained by the training unit 112 in the storage unit. In addition, the prediction unit 122 reads the trained prediction model from the storage unit, and inputs the material composition information notified from the material composition input unit 121 to the relevant trained prediction model, thereby successively predicting the phase ratio at each temperature within the predetermined temperature range. That is, the prediction unit 122 successively predicts the phase ratio within the predetermined temperature range for each of the predetermined temperature intervals.

The expression "successively predicting the phase ratio for each of the predetermined temperature intervals" means that the trained prediction model predicts the phase ratio at the (i+1)-th temperature, using the phase ratio predicted by the relevant trained prediction model for the temperatures to the i-th temperature (i is an integer greater than or equal to 1), for example.

As described above, in the present embodiment, the prediction unit 122 has a configuration for successively predicting the phase ratio at each temperature (that is, a configuration for processing the phase ratio at each temperature as the time series data by a recurrent neural network). Thus, the prediction unit 122 can perform a prediction which reflects prediction data of the phase ratio in the adjacent temperature region. As a result, compared to a case where a multilayer neural network is implemented (a case where the prediction result of the phase ratio in the adjacent temperature region is not reflected), for example, it is possible to reduce a decrease in a prediction accuracy of the phase ratio. That is, according to the prediction unit 122, it is possible to improve the prediction accuracy when predicting the phase ratio over the predetermined temperature range based on the material composition.

In addition, when the phase ratio within the predetermined temperature range is successively predicted for each of the predetermined temperature intervals, the prediction unit 122 performs a partial differentiation process on the predicted value of the phase specified in advance at each temperature, using the material composition of the material to be predicted. Hence, the prediction unit 122 can calculate a degree of modification (variation) of the phase specified in advance at each temperature in a case where the material composition is modified by a minute amount.

Moreover, in a heat map in which:
- each component of the material to be predicted is plotted along an abscissa (first axis); and
- each temperature within the predetermined temperature range is plotted along an ordinate (second axis),
the prediction unit 122 applies a color scheme to each of areas specified based on each component and based on each temperature, according to values obtained by performing the partial differentiation.

The display unit 123 displays the phase ratio at each temperature within the predetermined temperature range predicted by the prediction unit 122. The display unit 123 displays the phase ratio at each temperature within the predetermined temperature range predicted by the prediction unit 122, in a different color for each phase. However, the phase ratio at each temperature within the predetermined temperature range predicted by the prediction unit 122 may be displayed by changing a line type, such as a solid line, a dotted line, a one-dot chain line, or the like for each phase.

Further, the display unit 123 displays the heat map generated by the prediction unit 122. Accordingly, a designer of the material design can easily understand, based on the displayed heat map, how the material composition should be modified with respect to the phase ratio at each temperature within the predetermined temperature range that is displayed, in order to obtain a phase ratio close to a desired phase ratio.

As described above, according to the prediction device 120 of the first embodiment, when predicting the phase ratio based on the material composition information, the designer of the material design can easily understand the degree of impact of the modification of the material composition on the phase ratio, based on the heat map.

### <Hardware Configurations of Training Device and Prediction Device>

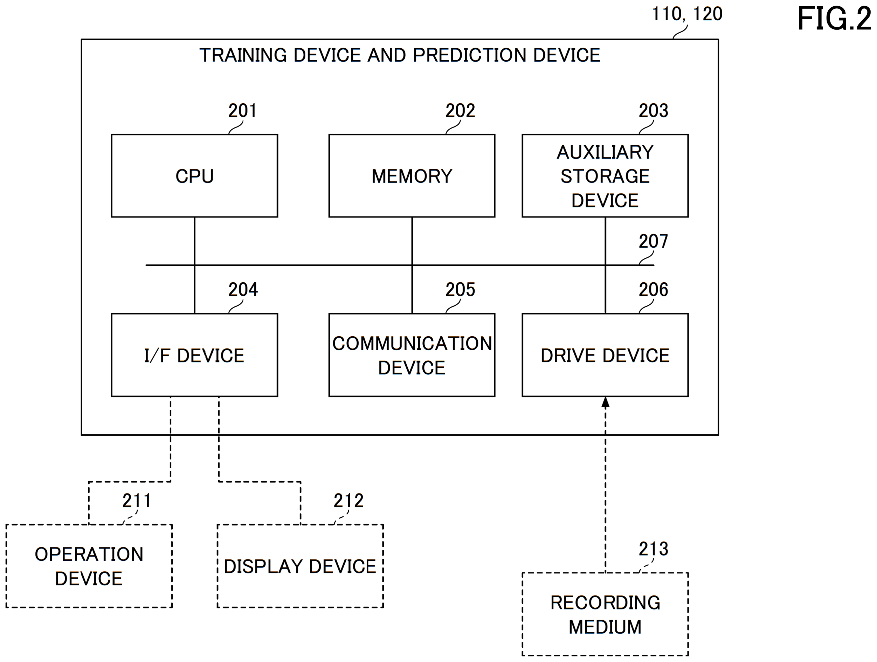

Next, hardware configurations of the training device 110 and the prediction device 120 will be described. Because the training device 110 and the prediction device 120 have the same hardware configuration, and the hardware configurations of the training device 110 and the prediction device 120 will be collectively described with reference to FIG. 2.

FIG. 2 is a diagram illustrating examples of hardware configurations of the training device and the prediction device. As illustrated in FIG. 2, the training device 110 and the prediction device 120 include a processor 201, a memory 202, an auxiliary storage device 203, an interface (I/F) device 204, a communication device 205, and a drive device 206. The hardware of the training device 110 and the hardware of the prediction device 120 are connected to each other via a bus 207.

The processor 201 includes various computing devices, such as a central processing unit (CPU), a graphics processing unit (GPU), or the like. The processor 201 reads various programs (for example, a training program, a prediction program, or the like) into the memory 202 and executes the programs.

The memory 202 includes a main storage device, such as a read only memory (ROM), a random access memory (RAM), or the like. The processor 201 and the memory 202 form a so-called computer, and the various functions described above are implemented by the computer when the processor 201 executes the various programs read into the memory 202.

The auxiliary storage device 203 stores various programs, and various data used when the various programs are executed by the processor 201. For example, the training data storage unit 113 is implemented by the auxiliary storage device 203. Alternatively, the storage unit that stores the trained prediction model is implemented by the auxiliary storage device 203.

The I/F device 204 is a connection device that connects to the operation device 211 and the display device 212, which are examples of user interface devices. The communication device 205 is a communication device for communicating with an external apparatus (not illustrated) via a network.

The drive device 206 is a device to which a recording medium 213 is loaded. The recording medium 213 includes a medium for optically, electrically, or magnetically recording information, such as a CD-ROM, a flexible disk, a magneto-optical disk, or the like. In addition, the recording medium 213 may include a semiconductor memory or the like for electrically recording information, such as a ROM, a flash memory, or the like.

The various programs installed in the auxiliary storage device 203 are installed by loading the recording medium 213 that is distributed into the drive device 206, and reading the various programs recorded in the recording medium 213 by the drive device 206, for example. Alternatively, the various programs installed in the auxiliary storage device 203 may be installed by being downloaded from the network via the communication device 205.

### <Configuration of Training Data, Input Data, and Ground Truth>

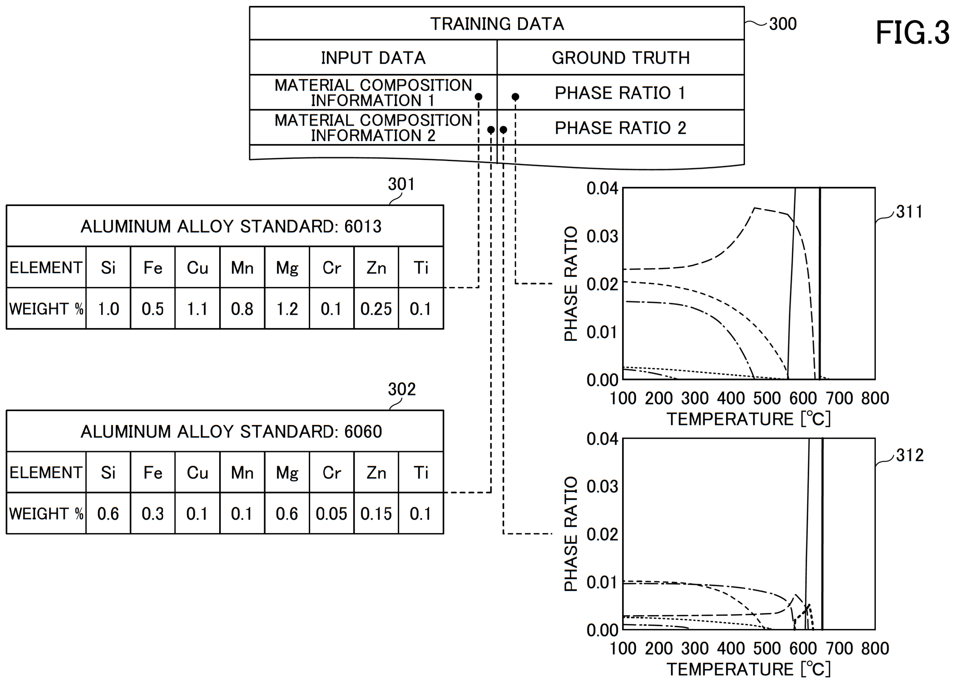

Next, a configuration of the training data, and specific examples of the input data and the ground truth included in the training data will be described. FIG. 3 is a diagram illustrating the configuration of training data and the specific examples of the input data and the ground truth.

As illustrated in FIG. 3, a training data 300 includes "input data" and "ground truth" as items of information.

The "input data" stores "material composition information 1", "material composition information 2", ... or the like, which are material composition information of different combinations. In FIG. 3, a reference numeral 301 indicates a specific example of the "material composition information 1", and represents an example of a weight % of each component (each of additive elements Si, Fe, Cu, Mn, Mg, Cr, Zn, and Ti) constituting "6013" which is a designation of the known aluminum alloy standard.

Similarly, a reference numeral 302 indicates a specific example of the "material composition information 2", and represents the weight % of each component (each of additive elements Si, Fe, Cu, Mn, Mg, Cr, Zn, and Ti) constituting "6060" which is a designation of a known aluminum alloy standard.

The "ground truth" stores "phase ratio 1", "phase ratio 2", ... or the like which are the phase ratios at respective temperatures within the predetermined temperature range (100°C to 800°C in the example of FIG. 3), associated with the "material composition information 1", "material composition information 2", ... or the like. In FIG. 3, a reference numeral 311 indicates a specific example of the "phase ratio 1", and a reference numeral 312 indicates a specific example of the "phase ratio 2", and in each case, the abscissa represents the temperature, and the ordinate represents the phase ratio.

The reference numeral 311 and the reference numeral 312 are results obtained by operating a simulator having a high prediction accuracy and a high cost for a long time, and in the present embodiment, these results are used as the ground truth.

The simulator described above refers to software for calculating the phase ratio at each temperature within the predetermined temperature range by a thermodynamic equilibrium calculation, and includes software, such as CaTCalc (registered trademark), MatCalc (registered trademark), or the like. Alternatively, the relevant simulator may include software, such as Termosuite (registered trademark), FactStage (registered trademark), Pandat (registered trademark), or the like. Alternatively, the relevant simulator may include software, such as MALT2 (registered trademark), Thermo-Calc (registered trademark), OpenCalphad (registered trademark), or the like.

### <Details of Each Unit of Training Device>

Next, details of each unit (the training data generation unit 111 and the training unit 112) of the training device 110 will be described.

### (1) Functional Configuration of Training Data Generation Unit:

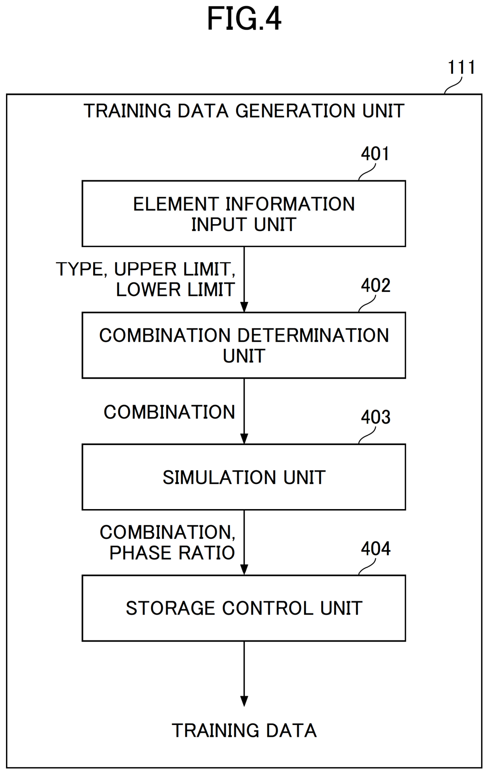

First, a functional configuration of the training data generation unit 111 will be described. FIG. 4 is a diagram illustrating an example of the functional configuration of the training data generation unit.

As illustrated in FIG. 4, the training data generation unit 111 includes an element information input unit 401, a combination determination unit 402, a simulation unit 403, and a storage control unit 404.

The element information input unit 401 receives an input of a type of an additive element to be added when generating a specific alloy, for example. The element information input unit 401 also receives an input of an upper limit value and a lower limit value of an addition amount (weight %) for each of the additive elements for which the input is received.

The combination determination unit 402 determines a plurality of combinations of the addition amounts of the respective additive elements, by randomly selecting the addition amounts of the respective additive elements for which the input is received, under restrictions of the upper limit value and the lower limit value. The combination of the addition amounts of the respective additive elements may be created at predetermined increments between the upper limit value and the lower limit value. Further, the combination determination unit 402 notifies the simulation unit 403 of the material composition information indicated by the plurality of combinations that are determined.

The simulation unit 403 calculates the phase ratio at each temperature within the predetermined temperature range by operating the simulator described above, for each of the plurality of material composition information notified from the combination determination unit 402. In addition, the simulation unit 403 notifies the storage control unit 404 of the plurality of material composition information, and the phase ratio at each temperature within the corresponding predetermined temperature range.

The storage control unit 404 generates training data in which the plurality of material composition information notified from the simulation unit 403 are set as the "input data" and the phase ratio at each temperature within the corresponding predetermined temperature range is set as the "ground truth", and stores the training data in the training data storage unit 113.

### (2) Functional Configuration of Training Unit:

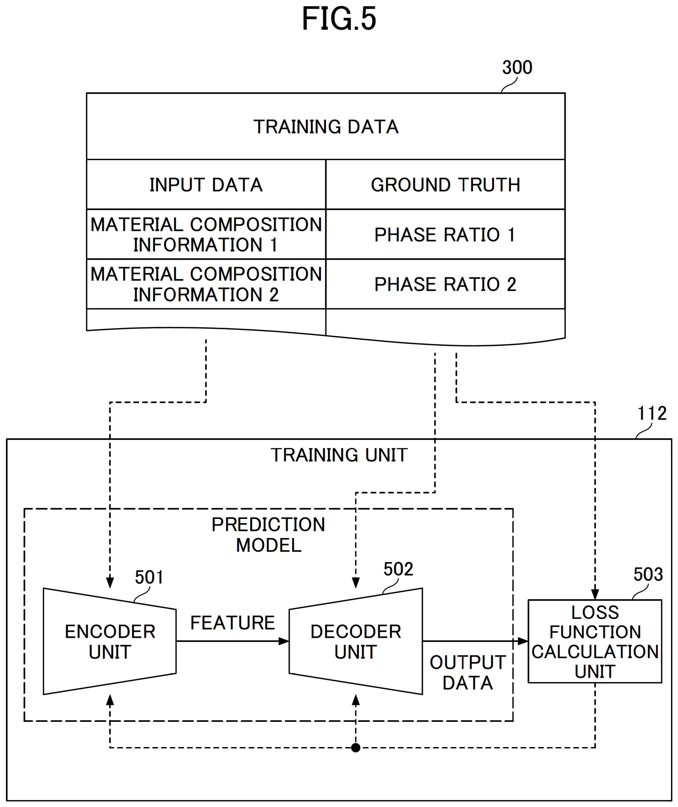

Next, a functional configuration of the training unit 112 will be described. FIG. 5 is a diagram illustrating an example of the functional configuration of the training unit.

As illustrated in FIG. 5, the training unit 112 includes a prediction model (surrogate model), and a loss function calculation unit 503.

As described above, the Seq2Seq with Attention mechanism or the Transformer is implemented as the prediction model, and the prediction model includes an encoder unit 501 and a decoder unit 502.

The encoder unit 501 outputs a feature in response to receiving the "material composition information 1", the "material composition information 2", ... or the like stored in the "input data" of the training data 300.

The decoder unit 502 successively outputs output data in response to receiving the feature output from the encoder unit 501. Specifically, the decoder unit 502 reads the "phase ratio 1", the "phase ratio 2", ... or the like stored in the "ground truth" of the training data 300. Then, the decoder unit 502 outputs the output data at the (i+1)-th temperature, using the phase ratios for the temperatures to the i-th temperature (that is, the ground truths for the temperatures to the i-th temperature). Accordingly, the decoder unit 502 can successively output the output data corresponding to the phase ratio at each temperature within the predetermined temperature range, based on the feature output from the encoder unit 501 in response to receiving the "material composition information 1", and the "ground truth" of the "phase ratio 1", for example. Similarly, the decoder unit 502 can successively output the output data corresponding to the phase ratio at each temperature within the predetermined temperature range, based on the feature output from the encoder unit 501 in response to receiving the "material composition information 2", and the "ground truth" of the "phase ratio 2", for example.

It is assumed that a softmax function is used in an output layer of the decoder unit 502. Thus, a plurality of values (that is, values indicating the ratio of each phase) included in the output data of the decoder unit 502 are in the range of 0 to 1, and a sum of the plurality of values (that is, a sum of the values indicating the ratio of each phase) becomes "1".

Hence, by using the softmax function in the output layer of the decoder unit 502, it is possible to successively output the output data corresponding to the phase ratio at each temperature within the predetermined temperature range, without performing additional calculation.

The loss function calculation unit 503 reads the "phase ratio 1", the "phase ratio 2", ... or the like stored in "ground truth" of the training data 300, and compares the read data with the output data at the (i+1)-th temperature output by the decoder unit 502.

In addition, the loss function calculation unit 503 calculates a plurality of types of losses when making the comparison, and calculates an integrated loss by performing a weighted addition of the plurality of calculated types of losses. Moreover, the loss function calculation unit 503 updates tmodel parameters of the encoder unit 501 and the decoder unit 502, based on the calculated integrated loss. Thus, a trained prediction model (including a trained encoder unit and a trained decoder unit) is generated.

### (3) Operation Example 1 of Training Unit:

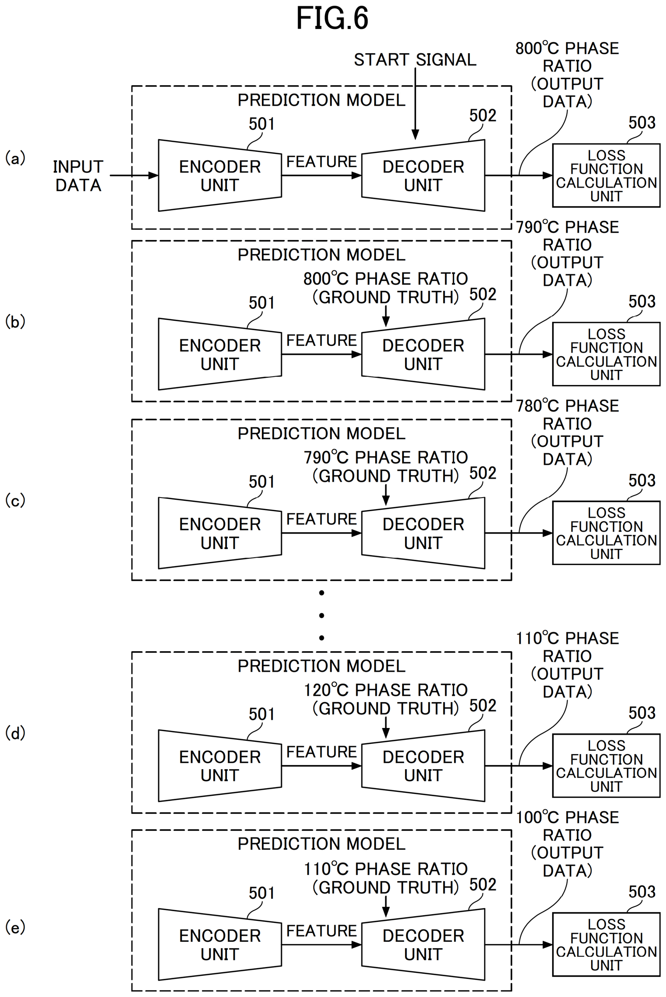

Next, an operation example of the training unit 112 (mainly an operation example of the prediction model between the prediction model and the loss function calculation unit) will be described. FIG. 6 is a first diagram illustrating an operation example of the training unit.

In FIG. 6, (a) illustrates a state in which the "input data" of the training data 300 is read and input to the encoder unit 501, and the feature is output from the encoder unit 501. Further, in FIG. 6,(a) illustrates a state in which the decoder unit 502 outputs the output data in response to a start signal input to the decoder unit 502. In the case of the example in (a) of FIG. 6, the output data output by the decoder unit 502 corresponds to the phase ratio at 800°C. The output data output from the decoder unit 502 (in this example, the output data corresponding to the phase ratio at 800°C) is notified to the loss function calculation unit 503. The start signal is a signal for starting the operation of the decoder unit 502, and in the present embodiment, an arbitrary value (for example, "000000" or the like) different from the output data output from the decoder unit 502 is input to the decoder unit 502 as the start signal.

In FIG. 6, (b) illustrates a state in which the feature output from the encoder unit 501 is input to the decoder unit 502, and the phase ratio at 800°C (the ground truth at 800°C) is input to the decoder unit 502, and thus, the decoder unit 502 outputs the output data. In the case of the example of (b) in FIG. 6, the output data output by the decoder unit 502 corresponds to the phase ratio at 790°C. The output data output from the decoder unit 502 (in this example, the output data corresponding to the phase ratio at 790°C) is notified to the loss function calculation unit 503.

In FIG. 6, (c) illustrates a state in which the feature output from the encoder unit 501 is input to the decoder unit 502, and the phase ratio for the temperatures to 790°C (the ground truth for the temperatures to 790°C) is input to the decoder unit 502, and thus, the decoder unit 502 outputs the output data. However, in (c) of FIG. 6, only the ground truth at 790°C is illustrated for the sake of convenience due to limited space (actually, the ground truth at 800°C and the ground truth at 790°C are input to the decoder unit 502 with weighting).

In the example illustrated in (c) of FIG. 6, the output data output from the decoder unit 502 corresponds to the phase ratio at 780°C. The output data output from the decoder unit 502 (the output data corresponding to the phase ratio at 780°C) is notified to the loss function calculation unit 503.

In FIG. 6, (d) illustrates a state in which the feature output from the encoder unit 501 is input to the decoder unit 502, and the phase ratio for the temperatures to 120°C (the ground truth for the temperatures to 120°C) is input to the decoder unit 502, and thus, the decoder unit 502 outputs the output data. However, in (d) of FIG. 6, only the ground truth at 120°C is illustrated for the sake of convenience due to limited space (actually, the ground truths at 800°C to 120°C are input to the decoder unit 502 with weighting).

In the example illustrated in (d) of FIG. 6, the output data output from the decoder unit 502 corresponds to the phase ratio at 110°C. The output data output from the decoder unit 502 (the output data corresponding to the phase ratio at 110°C) is notified to the loss function calculation unit 503.

In FIG. 6, (e) illustrates a state in which the feature output from the encoder unit 501 is input to the decoder unit 502, and the phase ratio for the temperatures to 110°C (the ground truth for the temperatures to 110°C) is input to the decoder unit 502, and thus, the decoder unit 502 outputs the output data. However, in (e) of FIG. 6, only the ground truth at 110°C is illustrated for the sake of convenience due to limited space (actually, the ground truths at 800°C to 110°C are input to the decoder unit 502 with weighting).

In the example illustrated in (e) of FIG. 6, the output data output from the decoder unit 502 corresponds to the phase ratio at 100°C. The output data output from the decoder unit 502 (the output data corresponding to the phase ratio at 100°C) is notified to the loss function calculation unit 503.

### (4) Operation Example 2 of Training Unit:

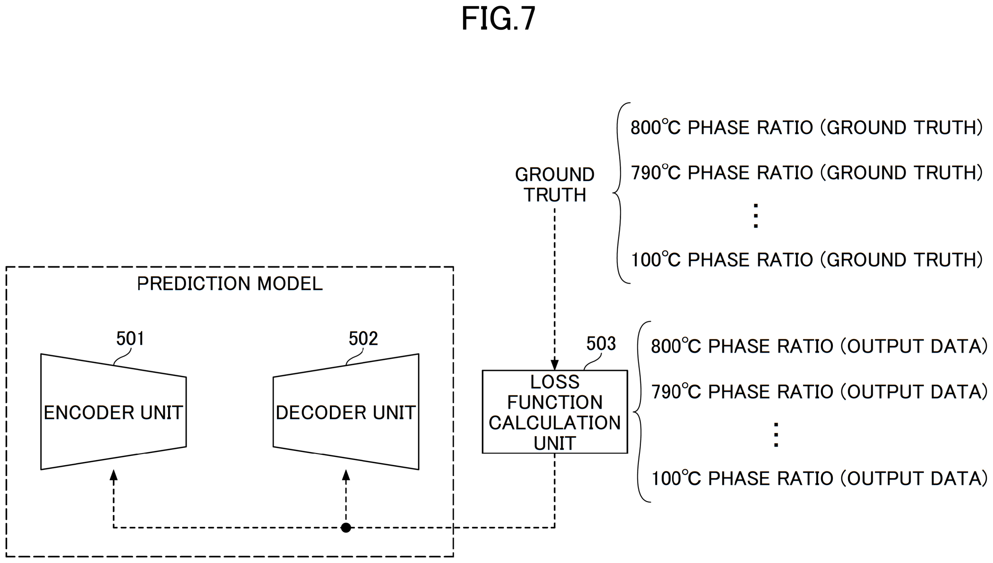

Next, an operation example of the training unit 112 (mainly, an operation example of the loss function calculation unit 503 between the prediction model and the loss function calculation unit) will be described. FIG. 7 is a second diagram illustrating the operation example of the training unit. As illustrated in FIG. 7, the loss function calculation unit 503 reads the "ground truth" of the training data 300. The example illustrated in FIG. 7 illustrates a state in which:
- the phase ratio at 800°C (the ground truth);
- the phase ratio at 790°C (the ground truth); ...
- the phase ratio at 100°C (the ground truth), which are the phase ratio at each temperature within the predetermined temperature range included in the "phase ratio 1".

In the example of FIG. 7 illustrates a state in which, as a result of successively outputting the output data from the decoder unit 502, based on the feature output from the encoder unit 501 in response to the input of the "material composition information 1":
- the phase ratio at 800°C (the output data);
- the phase ratio at 790°C (the output data); ...
- the phase ratio at 100°C (the output data),
are held in the loss function calculation unit 503.

In addition, the example of FIG. 7 illustrates a state in which the loss function calculation unit 503 compares the phase ratio (the ground truth) at each of the temperatures of 100°C to 800°C with the phase ratio (the output data) at each of the temperatures of 100°C to 800°C, and calculates the integrated loss. The example of FIG. 7 illustrates a state in which the loss function calculation unit 503 updates the model parameters of the encoder unit 501 and the decoder unit 502, based on the calculated integrated loss. The functional configuration of the loss function calculation unit 503 for calculating the integrated loss will be described below in more detail.

### (5) Details of Functional Configuration of Loss Function Calculation Unit:

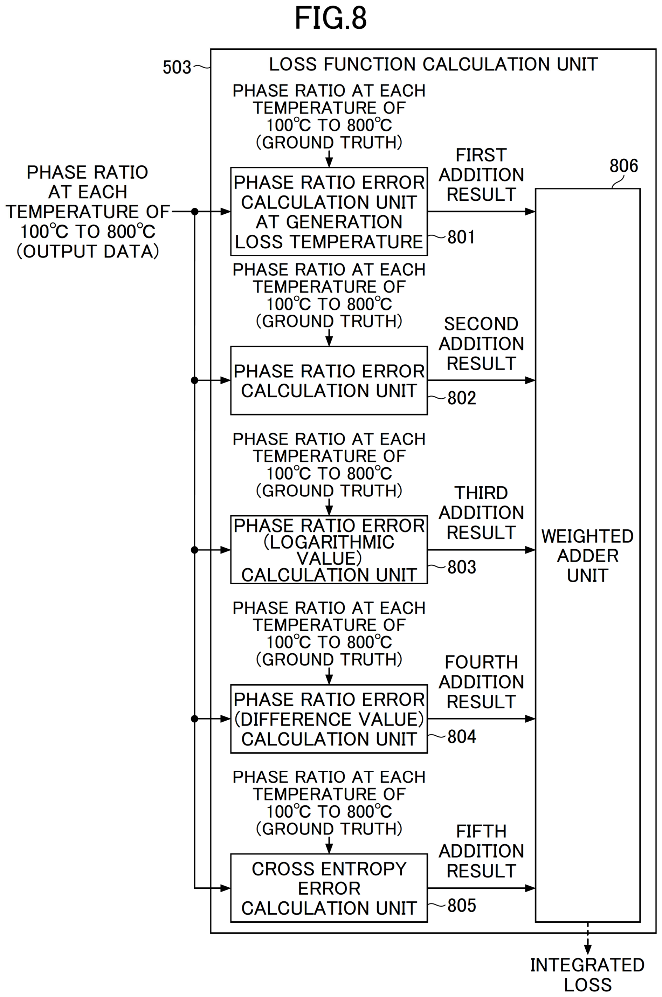

FIG. 8 is a diagram illustrating an example of a method of calculating a loss by the loss function calculation unit. As illustrated in FIG. 8, the loss function calculation unit 503 includes, as functions for calculating a plurality of types of losses:
- a phase ratio error calculation unit 801 at a generation/disappearance temperature;
- a phase ratio error calculation unit 802;
- a phase ratio error (logarithmic value) calculation unit 803;
- a phase ratio error (difference value) calculation unit 804;
- a cross entropy error calculation unit 805; and
- a weighted adder unit 806.

The phase ratio error calculation unit 801 at the generation/loss temperature compares the phase ratio (the ground truth) at each of the temperatures of 100°C to 800°C and the phase ratio (the output data) at each of the temperatures of 100°C to 800°C, with respect to the temperature at which the curve of each phase ratio becomes 0 in the ground truth of the training data. Thus, the phase ratio error calculation unit 801 at the generation/loss temperature adds the error between the phase ratio specified based on the output data and the phase ratio specified based on the training data, at the temperature at which each phase specified based on the training data is generated or lost, for all of the phases. In addition, the phase ratio error calculation unit 801 at the generation/loss temperature outputs an addition result (a first addition result).

The phase ratio error calculation unit 802 compares the phase ratio (the ground truth) at each of the temperatures of 100°C to 800°C with the phase ratio (the output data) at each of the temperatures of 100°C to 800°C. Accordingly, the phase ratio error calculation unit 802 adds the error between the phase ratios at each of the temperatures for the predetermined temperature range, and outputs an addition result (a second addition result).

The phase ratio error (logarithmic value) calculation unit 803 compares the phase ratio (the ground truth) at each of the temperatures of 100°C to 800°C with the phase ratio (the output data) at each of the temperatures of 100°C to 800°C. Accordingly, the phase ratio error (logarithmic value) calculation unit 803 adds the error between logarithmic values of the phase ratios at each of the temperatures for the predetermined temperature range, and outputs an addition result (a third addition result).

The phase ratio error (difference value) calculation unit 804 compares the phase ratio (the ground truth) at each of the temperatures of 100°C to 800°C with the phase ratio (the output data) at each of the temperatures of 100°C to 800°C. Accordingly, the phase ratio error (difference value) calculation unit 804 adds the error between difference values of the phase ratios at adjacent temperatures for the predetermined temperature range, and outputs an addition result (a fourth addition result).

The cross entropy error calculation unit 805 compares the phase ratio (the ground truth) at each of the temperatures of 100°C to 800°C with the phase ratio (the output data) at each of the temperatures of 100°C to 800°C. Accordingly, the cross entropy error calculation unit 805 adds the error between ratios of the phase ratios at each of the temperatures for the predetermined temperature range, and outputs an addition result (a fifth addition result).

The weighted adder unit 806 calculates the integrated loss by weighting and adding the addition results (the first addition result through the fifth addition result) output from the phase ratio error calculation unit 801 at the generation/loss temperature through the cross entropy error calculation unit 805.

Accordingly, by calculating the plurality of types of losses and performing the weighted addition, the loss function calculation unit 503 can process the loss between the output data and the ground truth from various perspectives. As a result, the training unit 112 can appropriately update the model parameters when training the prediction model.

### <Flow of Training Process>

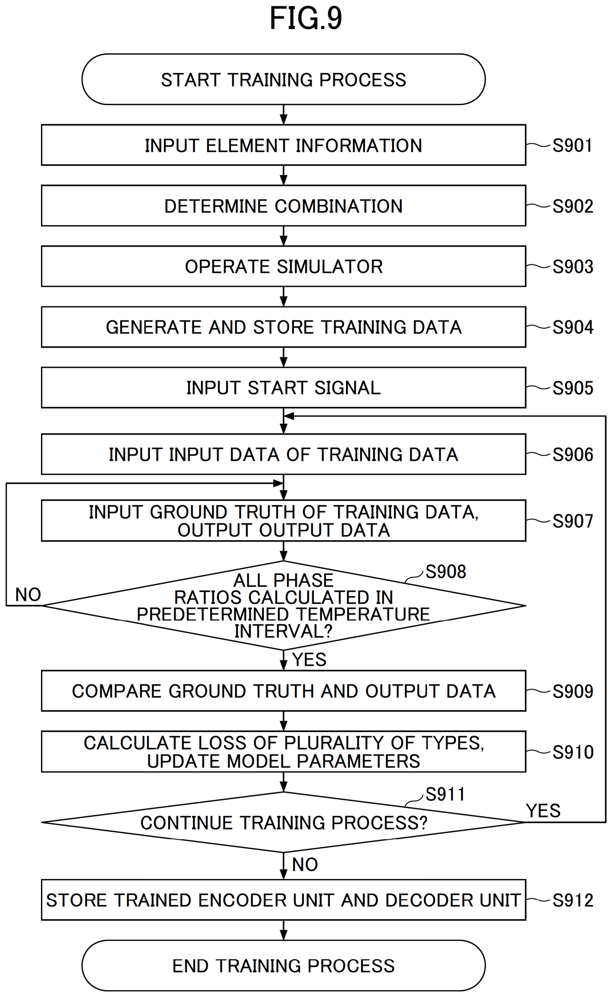

Next, a flow of a training process performed by the training device 110 will be described. FIG. 9 is a flow chart illustrating the flow of the training process.

In step S901, the training data generation unit 111 receives, as the element information, input of the types of additive elements to be added when generating a specific alloy, and the upper limit value and the lower limit value of the addition amount (weight %) of each of the additive elements.

In step S902, the training data generation unit 111 determines a plurality of combinations of the addition amounts (weight %) of the respective additive elements, by randomly selecting the addition amounts of the respective additive elements under the restrictions of the upper limit value and the lower limit value.

In step S903, the training data generation unit 111 operates the simulator for each of the material composition information indicated by the plurality of combinations that are determined, and calculates the phase ratio at each temperature within the predetermined temperature range.

In step S904, the training data generation unit 111 generates the training data, and stores the generated training data in the training data storage unit 113.

In step S905, the training unit 112 inputs the start signal to the decoder unit 502.

In step S906, the training unit 112 reads the "input data" of the training data, and inputs the input data to the prediction model.

In step S907, the training unit 112 successively inputs the phase ratios for the temperatures to the i-th temperature of the "ground truth" of the training data (that is, the ground truth for the temperatures to the i-th temperature), and successively outputs the (i+1)-th output data.

In step S908, the training unit 112 determines whether or not all of the output data corresponding to the phase ratio at each temperature within the predetermined temperature range are output. If it is determined in step S908 that there is a temperature for which the output data corresponding to the phase ratio is not output (when NO in step S908), the process returns to step S907.

On the other hand, if it is determined in step S908 that all of the output data corresponding to the phase ratio at each temperature within the predetermined temperature range are output (when YES in step S908), the process proceeds to step S909.

In step S909, the training unit 112 reads the "ground truth" of the training data, and compares the read ground truth with the output data.

In step S910, the training unit 112 calculates the plurality of types of losses, and performs the weighted addition of the plurality of types of losses that are calculated, in order to calculate the integrated loss. The training unit 112 updates the model parameters of the encoder unit 501 and the decoder unit 502, based on the calculated integrated loss.

In step S911, the training unit 112 determines whether or not to continue the training process. If it is determined in step S911 that the training process is to be continued (when YES in step S911), the process returns to step S906, and the same process is performed by reading the next "input data" of the training data.

On the other hand, if it is determined in step S911 that the training process is to be ended (when NO in step S911), the process proceeds to step S912.

In step S912, the training unit 112 stores the trained encoder unit and the trained decoder unit in the storage unit of the prediction device 120, as the trained prediction model.

### <Details of Each Unit of Prediction Device>

Next, each unit (the prediction unit 122 in this case) of the prediction device 120 will be described in more detail.

### (1) Functional Configuration of Prediction Unit:

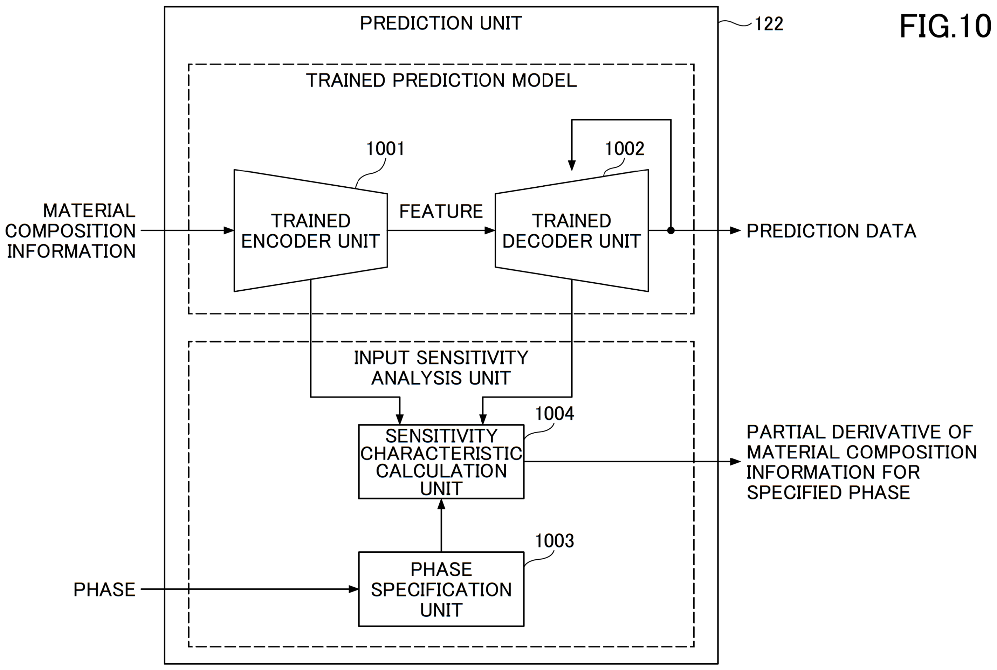

First, a functional configuration of the prediction unit 122 will be described. FIG. 10 is a diagram illustrating an example of the functional configuration of the prediction unit.

As illustrated in FIG. 10, the prediction unit 122 includes a trained encoder unit 1001 and a trained decoder unit 1002, which are already trained by the training unit 112. The trained encoder unit 1001 and the trained decoder unit 1002 form a trained prediction model.

The trained encoder unit 1001 calculates a feature in response to receiving the material composition information (the material composition information of the material to be predicted) notified from the material composition input unit 121, and outputs the calculated feature to the trained decoder unit 1002.

The trained decoder unit 1002 successively outputs the prediction data in response to receiving the feature output from the trained encoder unit 1001. Specifically, the trained decoder unit 1002 outputs the prediction data at the (i+1)-th temperature, using prediction data at the temperatures to the i-th temperature. Thus, the trained decoder unit 1002 can predict the phase ratio at each temperature within the predetermined temperature range, based on the feature output from the trained encoder unit 1001 in response to the input of the material composition information of the material to be predicted.

Further, as illustrated in FIG. 10, the prediction unit 122 further includes a phase specification unit 1003 and a sensitivity characteristic calculation unit 1004. The phase specification unit 1003 and the sensitivity characteristic calculation unit 1004 form an input sensitivity analysis unit.

The phase specification unit 1003 receives an input of a phase specified by the designer of the material design. The phase, specified by the designer of the material design, refers to a phase for which the designer of the material design wishes to understand an extent of a modification in advance, for each of the temperatures when the material composition is modified. The phase specification unit 1003 notifies the sensitivity characteristic calculation unit 1004 of the phase for which the input is received.

When outputting the prediction data at the (i+1)-th temperature, the sensitivity characteristic calculation unit 1004 performs a partial differentiation on the value predicted for the phase notified from the phase specification unit 1003, using the material composition information notified from the material composition input unit 121.

Specifically, the sensitivity characteristic calculation unit 1004 derives "the value obtained by partially differentiating the value at the (i+1)-th temperature predicted for the specified phase, using the material composition information" using:
- a partial derivative in each layer calculated by performing the process in each layer within the trained encoder unit 1001 during a period from the input of the feature to the output of the material composition information; and
- a partial derivative in each layer calculated by performing the process in each layer within the trained decoder unit 1002 during a period from the input of the feature to the output of the value predicted for the specified phase at the (i+1)-th temperature.

In addition, the sensitivity characteristic calculation unit 1004 notifies the display unit 123 (not illustrated in FIG. 10) of "the value obtained by partially differentiating the value at the (i+1)-th temperature predicted for the specified phase, using the material composition information".

The display unit 123 applies a color scheme to areas in the heat map, identified based on each component (each additive element) of the material to be predicted and the (i+1)-th temperature, according to the values obtained by the partial differentiation. Thus, the display unit 123 can generate and display the heat map of the predetermined temperature range by performing these processes over the predetermined temperature range.

### (2) Operation Example of Prediction Unit:

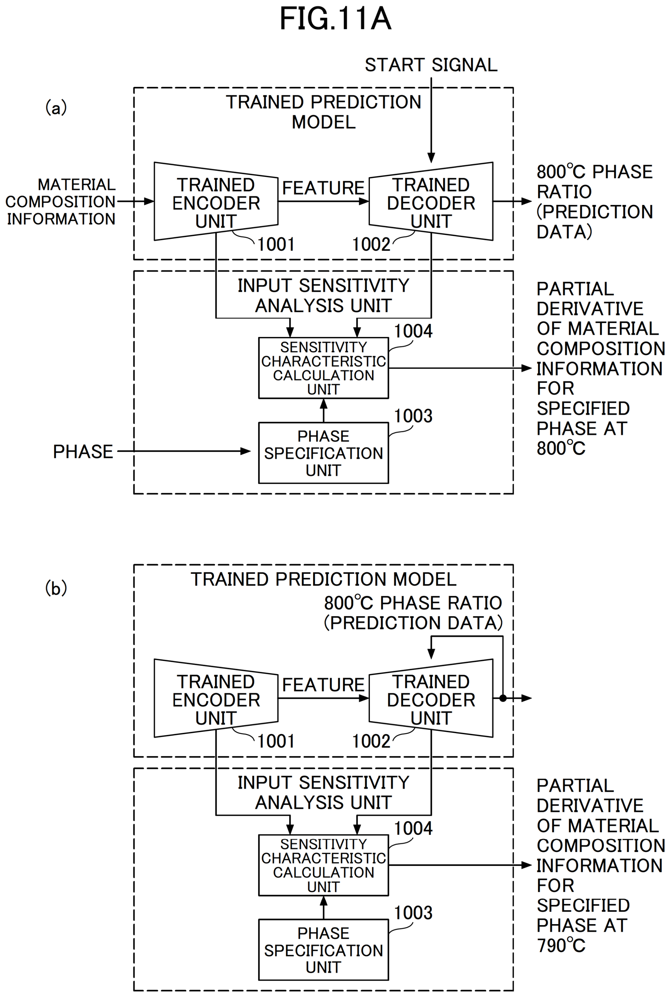

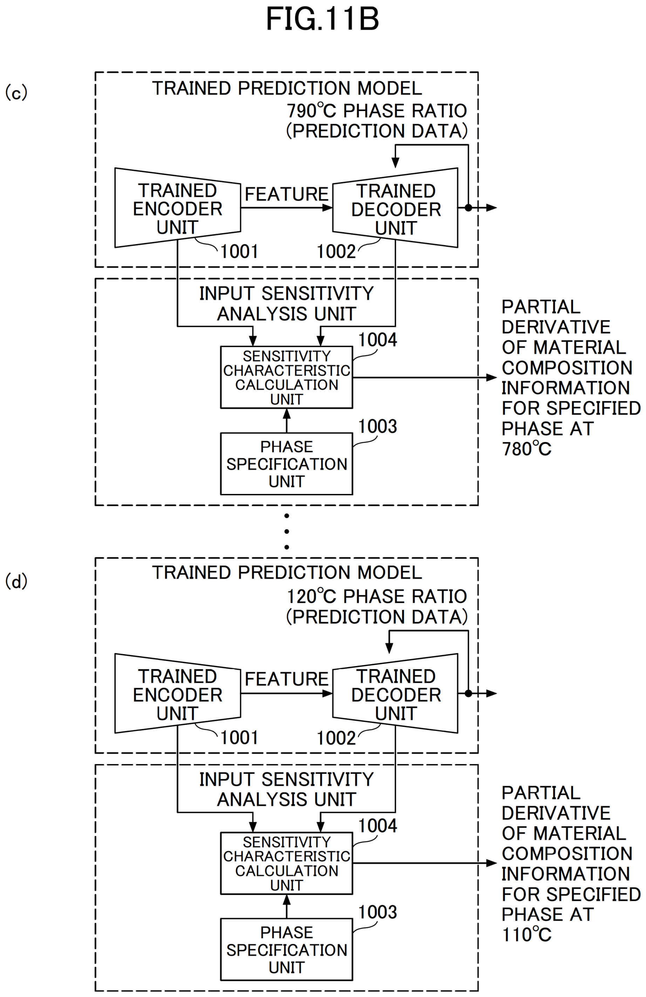

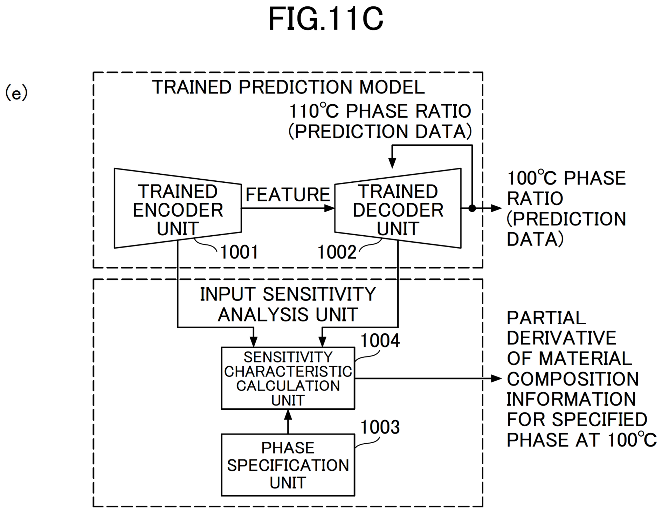

Next, an operation example of the prediction unit 122 will be described. FIG. 11A through FIG. 11C are first through third diagrams illustrating the operation examples of the prediction unit. Among FIG. 11A through FIG. 11C, (a) of FIG. 11A illustrates a state in which the material composition information of the material to be predicted is input to the trained encoder unit 1001, and thus, the feature is output from the trained encoder unit 1001. In addition, (a) of FIG. 11Aillustrates a state in which the feature output from the trained encoder unit 1001 is input to the trained decoder unit 1002, and the start signal is input to the trained decoder unit 1002, and thus, the prediction data (the phase ratio at 800°C) is output.

Moreover, (a) of FIG. 11Aillustrates a state in which "the value (the partial derivative) obtained by partially differentiating the value predicted for the specified phase, using the material composition information of the material to be predicted" is derived at 800°C, when the prediction data (the phase ratio at 800°C) is output.

(b) of FIG. 11A illustrates a state in which the feature output from the trained encoder unit 1001 is input to the trained decoder unit 1002, and the prediction data (the phase ratio at 800°C) is input to the trained decoder unit 1002. Thus, the trained decoder unit 1002 outputs the prediction data (the phase ratio at 790°C).

Further, (b) of FIG. 11A illustrates a state where "the value (the partial derivative) obtained by partially differentiating the value predicted for the specified phase, using the material composition information of the material to be predicted" is derived at 790°C, when the prediction data (the phase ratio at 790°C) is output.

(c) of FIG. 11B illustrates a state in which the feature output from the trained encoder unit 1001 is input to the trained decoder unit 1002, and the prediction value (the phase ratio at temperatures to 790°C) is input to the trained decoder unit 1002. In (c) of FIG. 11B, only the phase ratio at 790°C is illustrated for the sake of convenience due to limited space (actually, the phase ratio at 800°C and the phase ratio at 790°C are input to the trained decoder unit 1002 with weighting). Thus, the trained decoder unit 1002 outputs the prediction data (the phase ratio at 780°C).

In addition,(c) of FIG. 11B illustrates a state in which "the value (the partial derivative) obtained by partially differentiating the value predicted for the specified phase, using the material composition information of the material to be predicted" is derived at 780°C, when the prediction data (the phase ratio at 780°C) is output.

(d) of FIG. 11B illustrates a state in which the feature output from the trained encoder unit 1001 is input to the trained decoder unit 1002, and the prediction value (the phase ratio at temperatures to 120°C) is input to the trained decoder unit 1002. In (d) of FIG. 11B, only the phase ratio at 120°C is illustrated for the sake of convenience due to limited space (actually, the phase ratio at the temperatures of 800°C to 120°C are input to the trained decoder unit 1002 with weighting). Thus, the trained decoder unit 1002 outputs the prediction data (the phase ratio at 110°C).

Further, (d) of FIG. 11B illustrates a state in which "the value (the partial derivative) obtained by partially differentiating the value predicted for the specified phase, using the material composition information of the material to be predicted" is derived at 110°C, when the prediction data (the phase ratio at 110°C) is output.

(e) of FIG. 11C illustrates a state in which the feature output from the trained encoder unit 1001 is input to the trained decoder unit 1002, and the prediction value (the phase ratio to the temperature of 110°C) is input to the trained decoder unit 1002. In (e) of FIG. 11C , only the phase ratio at 110°C is illustrated for the sake of convenience due to limited space (actually, the phase ratio at the temperatures of 800°C to 110°C are input to the trained decoder unit 1002 with weighting). Thus, the example of (e) of FIG. 11C also illustrates the prediction data (the phase ratio at 100°C) output from the trained decoder unit 1002 in response to the input of the feature value and the prediction value (the phase ratio to the temperature of 110°C).

Further, (e) of FIG. 11C illustrates a state in which "the value (the partial derivative) obtained by partially differentiating the value predicted for the specified phase, using the material composition information of the material to be predicted" is derived at 100°C, when the prediction data (the phase ratio at 100°C) is output.

### <Flow of Prediction Process>

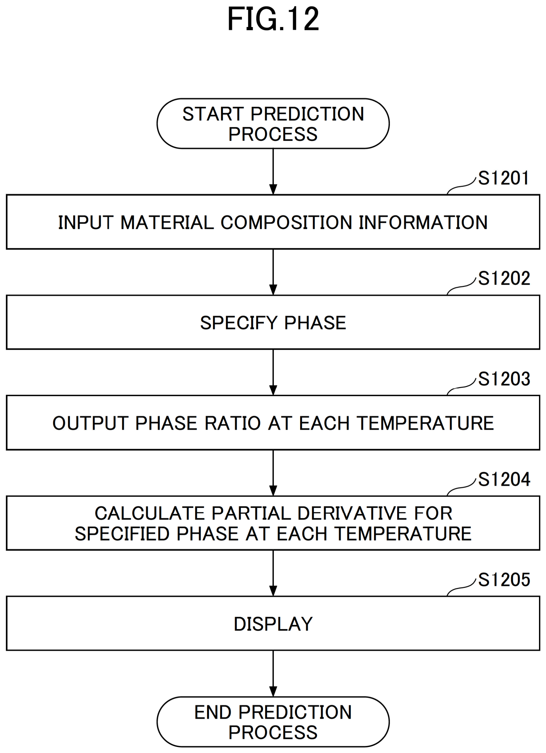

Next, a flow of a prediction process performed by the prediction device 120 will be described. FIG. 12 is a flow chart illustrating the flow of the prediction process.

In step S1201, the material composition input unit 121 receives an input of the material composition information of the material to be predicted.

In step S1202, the phase specification unit 1003 receives a specification of the phase.

In step S1203, the prediction unit 122 receives the start signal, and inputs the input material composition information to the trained prediction model, thereby predicting the phase ratio at each temperature within the predetermined temperature range.

In step S1204, the sensitivity characteristic calculation unit 1004 derives the partial derivative obtained by partially differentiating the value predicted for the specified phase at each temperature within the predetermined temperature range, using the material composition information.

In step S1205, the display unit 123 displays the predicted phase ratio at each temperature. Moreover, the display unit 123 displays the heat map for the specified phase.

### <Example of Predicted Phase Ratio>

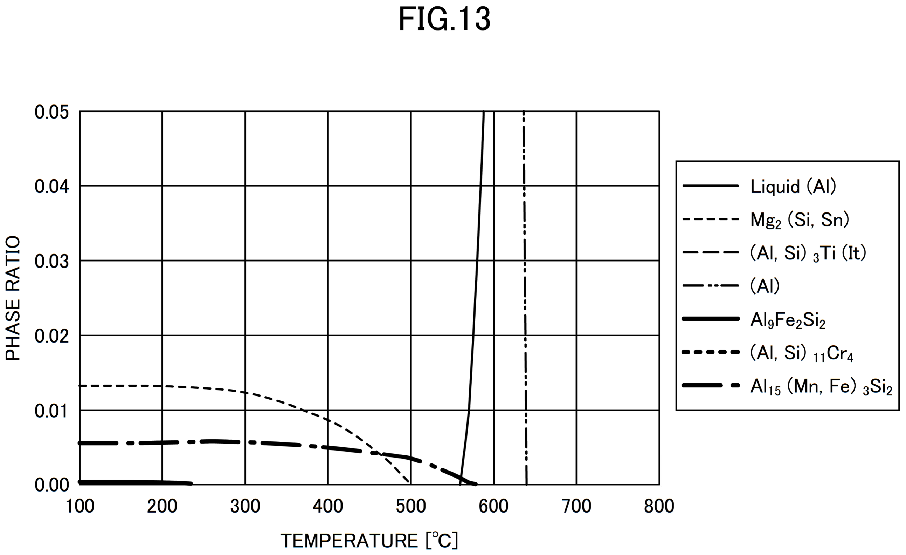

Next, a specific example of the phase ratio at each temperature within the predetermined temperature range predicted by the prediction unit 122 will be described. FIG. 13 is a diagram illustrating an example of the predicted phase ratio, and illustrates the prediction data for a case where the weight % of each additive element constituting a designation "6013" of the known aluminum alloy standard (in this example, the weight % of a minimum composition) is input as the material composition information of the material to be predicted.

When evaluating the prediction accuracy in the present embodiment, the output for a case where the simulator described above is operated with the input of the weight % of each additive element constituting the known aluminum alloy standard having the designation "6013" (in this example, the weight % of the minimum composition), is used as the ground truth. In addition, a means squared logarithmic error (MSLE) loss is used to calculate the loss with respect to the ground truth. In the case of FIG. 13, the MSLE loss between the prediction data and the ground truth was 2.45 × 10⁻⁵.

Accordingly, when predicting the phase ratio over the predetermined temperature range based on the material composition information:
- the phase ratio at each temperature is processed as time series data, using a recurrent neural network; and
- the loss is processed from various perspectives, using a plurality of types of loss functions,
and thus, the prediction device 120 according to the present embodiment can improve the prediction accuracy.

### <Example of Heat Map>

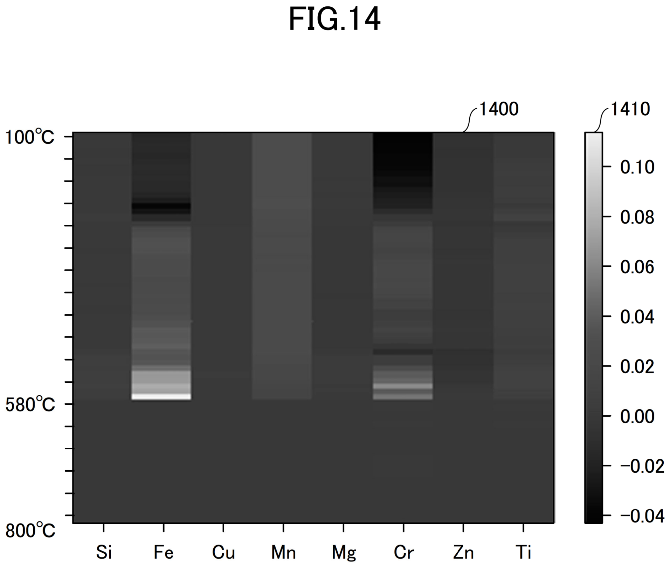

Next, a specific example of the heat map displayed by the display unit 123 will be described. FIG. 14 is a diagram illustrating an example of the heat map, and illustrates the heat map for a case where a phase = "Al₁₅(Mn, Fe)₃Si₂" is specified by the designer.

As illustrated in FIG. 14, in a heat map 1400, the abscissa represents each component (each additive element) of the material to be predicted. In the case of FIG. 14, Si, Fe, Cu, Mn, Mg, Cr, Zn, and Ti, which are the respective additive elements constituting the known aluminum alloy standard having the designation "6013", are arranged on the abscissa as the respective components (respective additive elements) of the material to be predicted.

As illustrated in FIG. 14, in the heat map 1400, the ordinate represents each temperature within the predetermined temperature range of 100°C to 800°C. In FIG. 14, a reference numeral 1410 denotes an example of the color scheme according to the partial derivative derived by the prediction unit 122. In the example of FIG. 14, the lighter the color is, the larger the degree of impact is on the designated phase (the larger the partial derivative is), and the darker the color is, the smaller the degree of impact is on the designated phase (the smaller the partial derivative is).

According to the example of FIG. 14, the designer of the material design can easily understand the impact on a temperature range of a specified phase when the weight % of an additive element is increased or decreased, such as:
- the phase = "Al₁₅(Mn, Fe)₃Si₂" greatly increases near 580°C, when the weight % of the additive element = "Fe" is increased;
- the phase = "Al₁₅ (Mn, Fe)₃Si₂" increases constantly from 100°C to 580°C, when the weight % of the additive element = "Mn" is increased; and
- the impact on the phase = "Al₁₅ (Mn, Fe)₃Si₂" is small even when the weight % of the additive element = "Si" is increased.

Accordingly, by configuring the material design system so that the degree of impact on the phase ratio due to the modification of the material composition can be understood in advance, the designer of the material design can perform an efficient material design. By using the configuration described above, the process can be performed in parallel with the output of the prediction data (the prediction of the phase ratio). For this reason, compared to a method of predicting the phase ratio within the predetermined temperature range while successively increasing or decreasing the weight % of all of the additive elements and comparing the predicted phase ratio with the phase ratio before the modification, for example, it is possible to greatly reduce a calculation load of the prediction device 120.

### <Verification of Heat Map>

Next, a verification result of verifying the calculation accuracy of the heat map 1400 illustrated in FIG. 14 in the following manner will be described.
- Material to be predicted: aluminum alloy (alloy standard "6013");
- Additive elements to be modified of the weight %: Si, Fe, Cu, Mn, Mg, Cr, Zn, Ti;
- Method for modifying the weight %: increase the weight % by 0.05 from a minimum composition weight % (Si = 0.6, Fe = 0.01, Cu = 0.6, Mn = 0.2, Mg = 0.8, Cr = 0.01, Zn = 0.01, Ti = 0.01, Zr = 0, B = 0, Bi = 0, Pb = 0, Al = 97.76);
- Predetermined temperature range: 100°C to 800°C;
- Specified phase: Al₁₅(Mn, Fe)₃Si₂; and
- Method of calculating data (difference value) used for verification: the values indicating the ratio of Al₁₅(Mn, Fe)₃Si₂ at each temperature before and after the modification are calculated by a simulator (Thermo-Calc (registered trademark)), and the difference value before and after the modification is calculated.

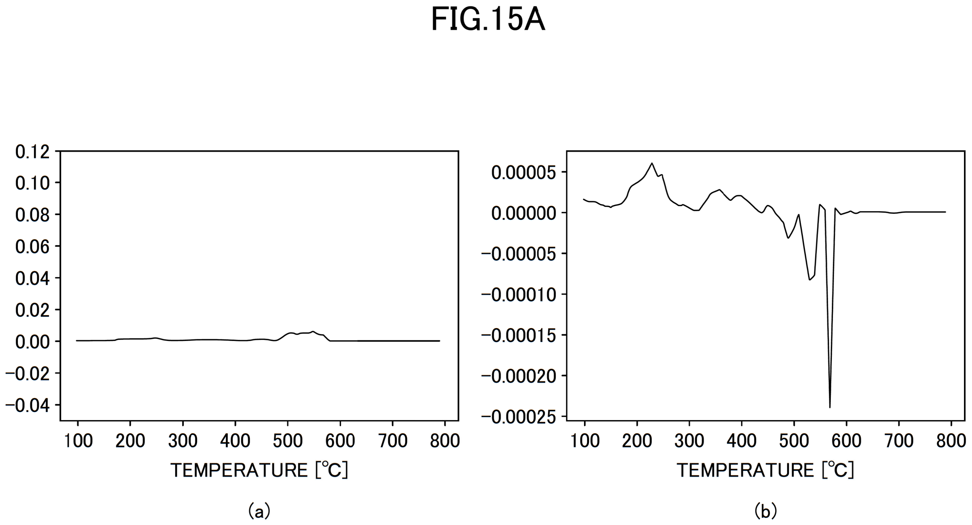

FIG. 15A through FIG. 15H are first through eighth diagrams illustrating examples of the verification result. FIG. 15A illustrates the difference before and after the modification calculated for a case where the weight % of Si is increased by a predetermined weight % (= 0.05) from the weight % (= 0.6) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but a range of the ordinate is narrower in (b)).

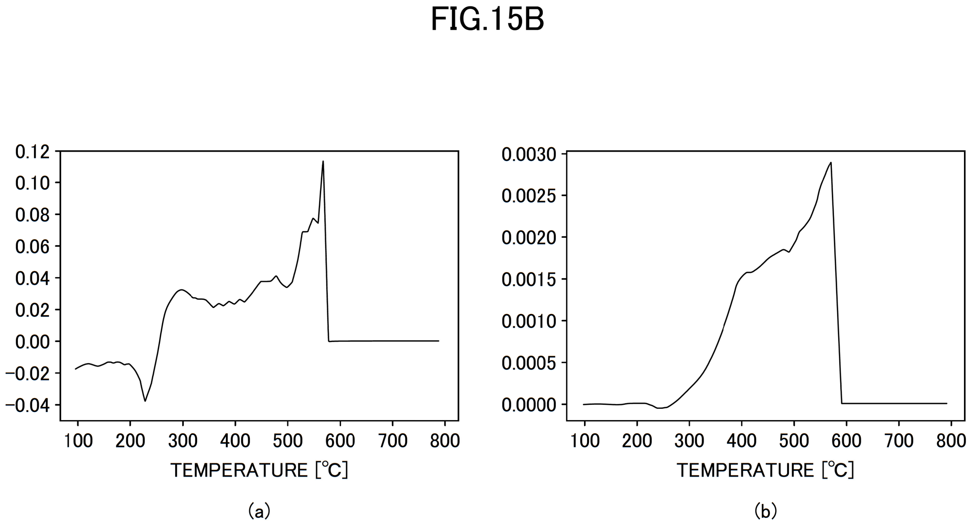

Similarly, FIG. 15B illustrates the difference before and after the modification calculated for a case where the weight % of Fe is increased by a predetermined weight % (= 0.05) from the weight % (= 0.01) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but the range of the ordinate is narrower in (b)).

When a transition of the graph from 100°C to 800°C illustrated in (a) of FIG. 15B is compared with a transition of the graph from 100°C to 800°C illustrated in (b) of FIG. 15B, the tendencies are generally the same. For example, the tendency that the difference value increases near 580°C is the same for the two graphs.

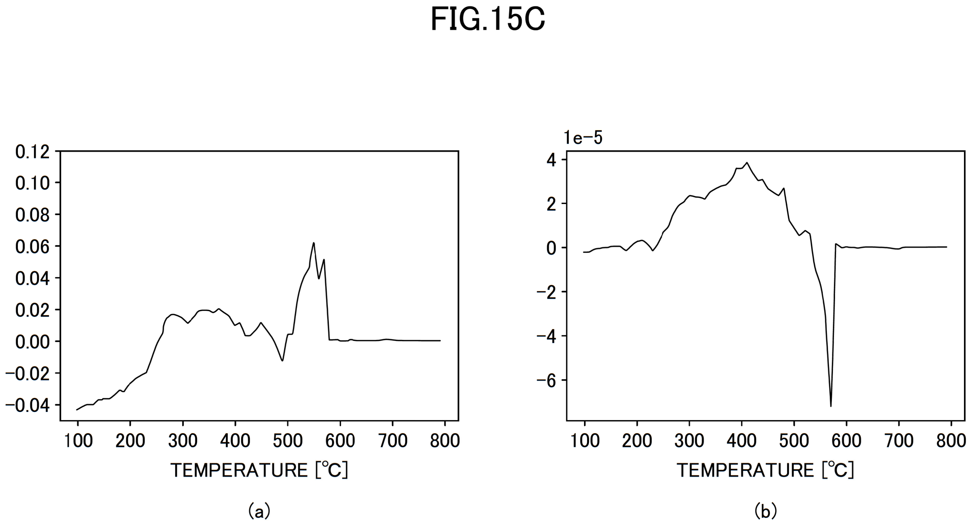

Similarly, FIG. 15C illustrates the difference before and after the modification calculated for a case where the weight % of Cu is increased by a predetermined weight % (= 0.05) from the weight % (= 0.6) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but the range of the ordinate is narrower in (b)).

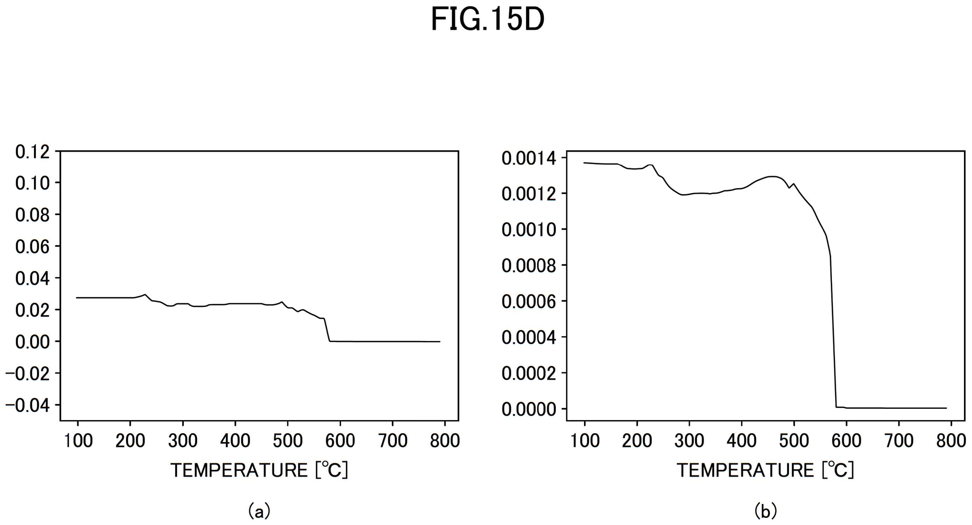

Similarly, FIG. 15D illustrates the difference before and after the modification calculated for a case where the weight % of Mn is increased by a predetermined weight % (= 0.05) from the weight % (= 0.2) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but the range of the ordinate is narrower in (b)).

When a transition of the graph from 100°C to 800°C illustrated in (a) of FIG. 15D is compared with a transition of the graph from 100°C to 800°C illustrated in (b) of FIG. 15D, the tendencies are generally the same. For example, the tendency that the value indicating the ratio of Al₁₅(Mn, Fe)₃Si₂ increases over the range of 100°C to 580°C is the same for the two graphs.

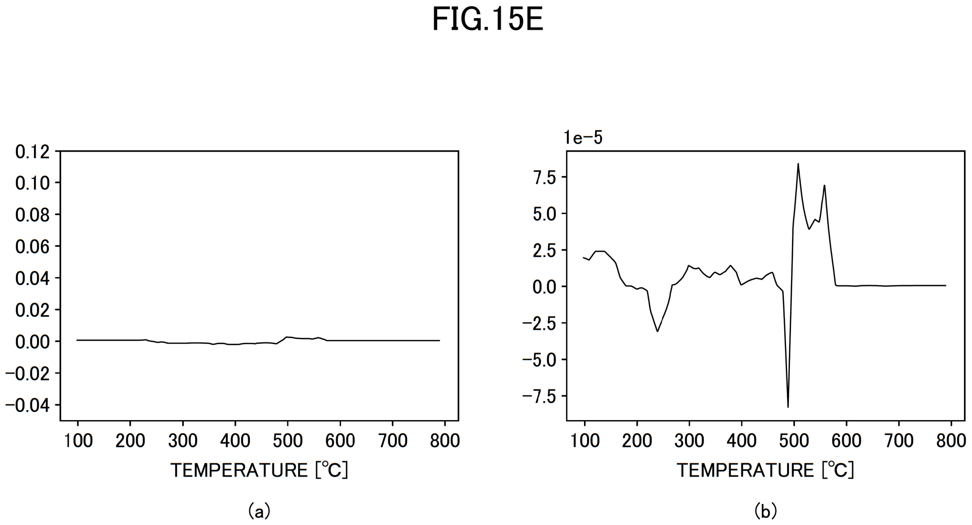

Similarly, (a) in FIG. 15E illustrates the difference before and after the modification calculated for a case where the weight % of Mg is increased by a predetermined weight % (= 0.05) from the weight % (= 0.8) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but the range of the ordinate is narrower in (b)).

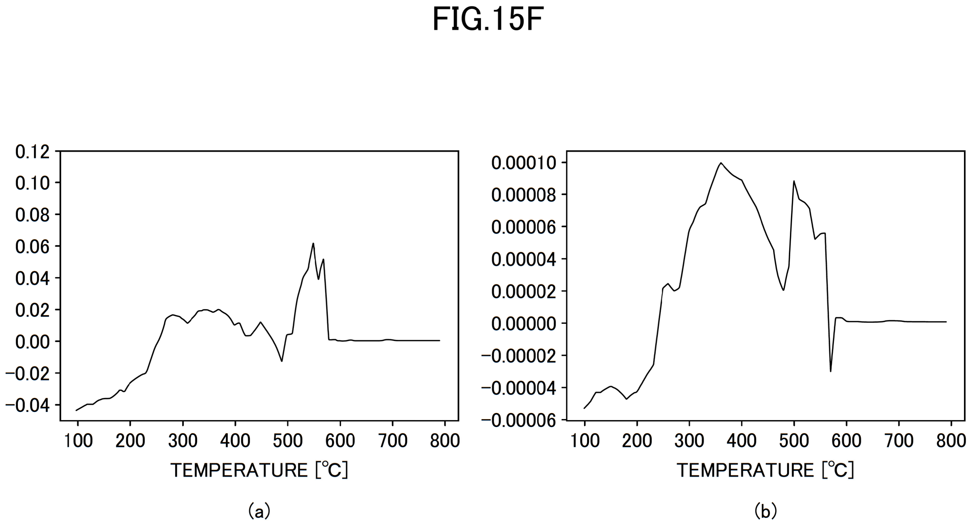

Similarly, FIG. 15F illustrates the difference before and after the modification calculated for a case where the weight % of Cr is increased by a predetermined weight % (= 0.05) from the weight % (= 0.01) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but the range of the ordinate is narrower in (b)).

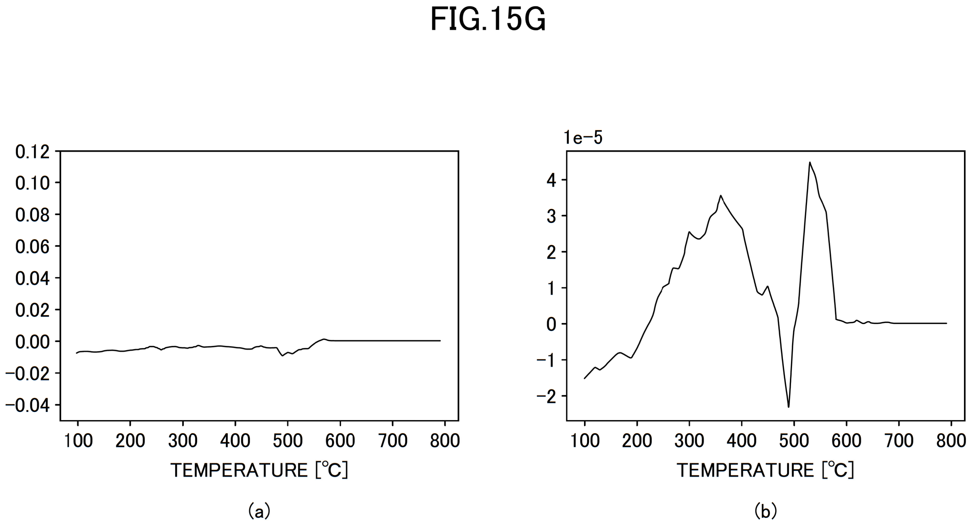

Similarly, FIG. 15G illustrates the difference before and after the modification calculated for a case where the weight % of Zn is increased by a predetermined weight % (= 0.05) from the weight % (= 0.01) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but the range of the ordinate is narrower in (b)).

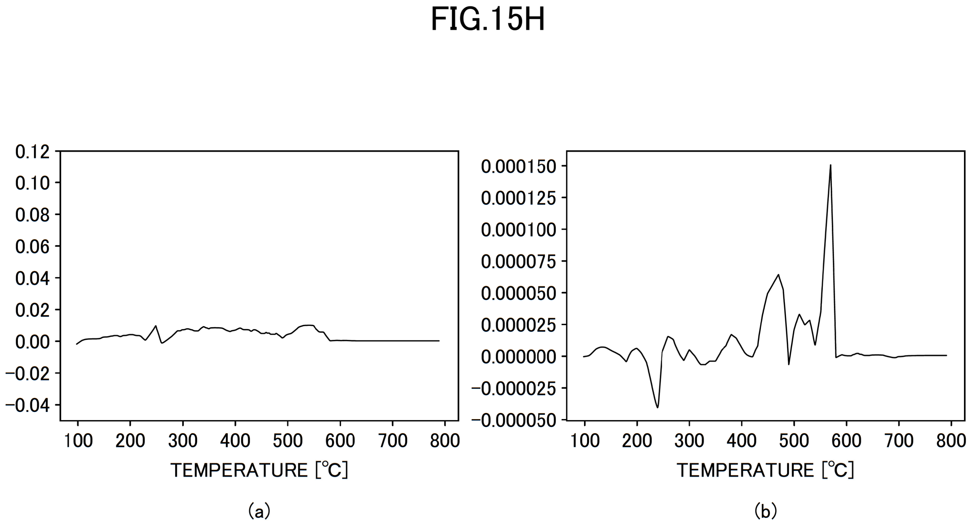

Similarly, FIG. 15H illustrates the difference before and after the modification calculated for a case where the weight % of Ti is increased by a predetermined weight % (= 0.05) from the weight % (= 0.01) of the minimum composition, and the weight % of Al is decreased by a predetermined weight % (= 0.05), where:
- (a) illustrates the case where the difference value between the values indicating the ratio of Al₁₅(Mn, Fe)₃Si₂ at the respective temperatures before and after the modification is calculated from the partial derivative described above; and
- (b) illustrates the case where the difference value between values indicating the ratio of Al₁₅(Mn, Fe)₃Si₂ at the respective temperatures before and after the modification is calculated by the simulator (Thermo-Calc (registered trademark)) (but the range of the ordinate is narrower in (b)).

As described above, it may be regarded that the heat map 1400 illustrated in FIG. 14 reproduces the tendency of the additive elements (for example, Fe and Mn) for which the difference before and after the modification is large.

### <Conclusion>

As is clear from the above description, the prediction device 120 according to the first embodiment includes the following features:
- The storage unit configured to store a trained prediction model that is trained using training data in which the material composition of the material to be trained, and the phase ratio of the material to be trained at each temperature within the predetermined temperature range, are associated with each other.
- When the phase ratio of the material to be predicted at each temperature within the predetermined temperature range is predicted by inputting the material composition of the material to be predicted to the trained prediction model, the predicted value of the phase specified in advance at each temperature is partially differentiated using the material composition of the material to be predicted.
- In the heat map in which each component of the material to be predicted is arranged on the first axis and each temperature within the predetermined temperature range is arranged on the second axis, each area specified based on each component and based on each temperature is displayed in the color scheme according to the value obtained by the partial differentiation.

Thus, according to the first embodiment, it is possible to easily understand the degree of impact on the phase ratio caused by the modification of the material composition.

### [Second Embodiment]

In the first embodiment, the color scheme of the heat map is a color scheme indicated by the reference numeral 1410 in FIG. 14, but a color scheme other than the color scheme indicated by the reference numeral 1410 may be employed. Moreover, the heat map may be represented in a display mode other than the color scheme.

Further, the first embodiment describes a case where the calculated partial derivative is represented using the heat map, so that the designer can easily understand the weight % of which of the additive elements can be increased or decreased to impact the phase ratio of which temperature range. However, the method of expressing the calculated partial derivative is not limited to using the heat map. For example, in a case where it is desirable to understand which temperature within the predetermined temperature range has a large degree of impact, a graph representing the transition of the degree of impact due to the difference in temperature may be displayed in place of the heat map.

In addition, although the first embodiment describes the alloy composition indicating the weight % of the additive element (another metal element or a non-metal element) with respect to the metal element included in the alloy as a specific example of the material composition, the material composition is not limited to the alloy composition. For example, a chemical composition may indicate the weight % of each chemical component included in a substance other than the alloy.

Moreover, in the first embodiment, the Seq2Seq with Attention mechanism or the Transformer is implemented as the trained prediction model. However, the trained prediction model is not limited to such, and other architectures may be implemented as long as the architecture can process the time series data, that are the data at predetermined time intervals. For example, a recurrent neural network (RNN), a bidirectional RNN, a Seq2Seq, or the like may be implemented. Alternatively, a gated recurrent unit (GRU), a long short term memory (LSTM), or the like may be implemented.

Further, although the training device 110 and the prediction device 120 are configured as separate devices in the first embodiment, the training device 110 and the prediction device 120 may be integrated and configured as an integrated device.

The present invention is not limited to the configurations or the like described in the embodiments, and combinations with other elements or the like are possible. These modifications can be made without departing from the scope of the present invention, and can be appropriately determined according to the form of application.

This application is based upon and claims priority to Japanese Patent Application No. 2022-211219 filed on December 28, 2022, the entire contents of which are incorporated herein by reference.

### DESCRIPTION OF REFERENCE NUMERALS

100: prediction system
110: training device
111: training data generation unit
112: training unit
120: prediction device
121: material composition input unit
122: prediction unit
123: display unit
300: training data
401: element information input unit
402: combination determination unit
403: simulation unit
404: storage control unit
501: encoder unit
502: decoder unit
503: loss function calculation unit
801: phase ratio error calculation unit at
generation/loss temperature
802: phase ratio error calculation unit
803: phase ratio error (logarithmic value) calculation unit
804: phase ratio error (difference value) calculation unit
805: cross entropy error calculation unit
806: weighted adder unit
1001: trained encoder unit
1002: trained decoder unit
1003: phase specification unit
1004: sensitivity characteristic calculation unit
1400: heat map

## Claims

1. A prediction device comprising:
a storage unit configured to store a trained model that is trained using training data in which a material composition of a material to be trained, and a phase ratio of the material to be trained at each temperature within a predetermined temperature range, are associated with each other, the trained model being configured to predict a phase ratio at an (i+1)-th temperature using phase ratios predicted by the trained model for temperatures to an i-th temperature (i is an integer greater than or equal to 1) within the predetermined temperature range, wherein:
a value predicted for a phase specified in advance is partially differentiated using a material composition of a material to be predicted at each temperature, when the phase ratio of the material to be predicted at each temperature within the predetermined temperature range is predicted by inputting the material composition of the material to be predicted to the trained model, and
each area specified based on each component and based on each temperature is displayed in a display mode according to the partially differentiated value, in a heat map in which each component of the material to be predicted is arranged on a first axis and each temperature within the predetermined temperature range is arranged on a second axis.

2. The prediction device as claimed in claim 1, wherein an architecture capable of processing time series data which are data at predetermined time intervals is applied to the trained model, and the trained model predicts a phase ratio for each predetermined temperature interval based on the material composition of the material to be predicted.

3. The prediction device as claimed in claim 2, wherein the trained model is any one of an RNN, a bidirectional RNN, a Seq2Seq, a Seq2Seq with Attention mechanism, a GRU, an LSTM, and a Transformer.

4. The prediction device as claimed in claim 3, wherein the trained model includes:
an encoder unit configured to output a feature, in response to an input of the material composition of the material to be predicted, and
a decoder unit configured to predict the phase ratio at the (i+1)-th temperature, in response to an input of the output feature and predicted phase ratios for the temperatures to the i-th temperature.

5. The prediction device as claimed in any one of claims 1 to 4, wherein the phase ratio is a phase ratio in a thermodynamic equilibrium state.

6. The prediction device as claimed in any one of claims 1 to 5, wherein:
the value predicted for the phase specified in advance is partially differentiated using the material composition of the material to be predicted, when predicting the phase ratio at the (i+1)-th temperature, and
each area specified based on each component and the (i+1)-th temperature in the heat map is displayed in a display mode according to the partially differentiated value.

7. A prediction method to be implemented in a computer for a trained model that is trained using training data in which a material composition of a material to be trained, and a phase ratio of the material to be trained at each temperature within a predetermined temperature range, are associated with each other, the trained model being configured to predict a phase ratio at an (i+1)-th temperature using phase ratios predicted by the trained model for temperatures to an i-th temperature (i is an integer greater than or equal to 1) within the predetermined temperature range, the prediction method comprising the steps of:
predicting, by the computer, a value predicted for a phase specified in advance is partially differentiated using a material composition of a material to be predicted at each temperature, when the phase ratio of the material to be predicted at each temperature within the predetermined temperature range is predicted by inputting the material composition of the material to be predicted to the trained model, and
displaying, by the computer, each area specified based on each component and based on each temperature in a display mode according to the partially differentiated value, in a heat map in which each component of the material to be predicted is arranged on a first axis and each temperature within the predetermined temperature range is arranged on a second axis.

8. A prediction program for causing a computer to implement a trained model that is trained using training data in which a material composition of a material to be trained, and a phase ratio of the material to be trained at each temperature within a predetermined temperature range, are associated with each other, the trained model being configured to predict a phase ratio at an (i+1)-th temperature using phase ratios predicted by the trained model for temperatures to an i-th temperature (i is an integer greater than or equal to 1) within the predetermined temperature range, the prediction program causing the computer to perform the steps of:
predicting a value predicted for a phase specified in advance is partially differentiated using a material composition of a material to be predicted at each temperature, when the phase ratio of the material to be predicted at each temperature within the predetermined temperature range is predicted by inputting the material composition of the material to be predicted to the trained model, and
displaying each area specified based on each component and based on each temperature in a display mode according to the partially differentiated value, in a heat map in which each component of the material to be predicted is arranged on a first axis and each temperature within the predetermined temperature range is arranged on a second axis.
